Europäisches Patentamt

European Patent Office

Office européen des brevets

(19)

(11) Veröffentlichungsnummer: **0 446 799 A2**

(12) **EUROPÄISCHE PATENTANMELDUNG**

(21) Anmeldenummer: 91103559.0

(22) Anmeldetag: 08.03.91

(51) Int. Cl.5: **A61B 17/36**

(30) Priorität: **15.03.90 DE 4008217**

(43) Veröffentlichungstag der Anmeldung:
**18.09.91 Patentblatt 91/38**

(84) Benannte Vertragsstaaten:
**DE FR GB**

(71) Anmelder: **Richard Wolf GmbH**
**Pforzheimer Strasse 32**
**W-7134 Knittlingen(DE)**

(72) Erfinder: **Kolb, Achim**
**Turbanstrasse 2/1**
**W-7518 Bretten(DE)**
Erfinder: **Baier, Manfred**
**Unterer Steinweg 54/2**
**W-7134 Knittlingen(DE)**
Erfinder: **Müller, Klaus**
**Knittlinger Strasse 44**
**W-7134 Knittlingen(DE)**

(74) Vertreter: **Vollmann, Heiko, Dipl.-Ing. et al**
**Patentanwälte Wilcken & Wilcken,**
**Musterbahn 1**
**W-2400 Lübeck 1(DE)**

(54) **Vorrichtung zur Gasspülung eines Wellenleiters für CO2-Laser.**

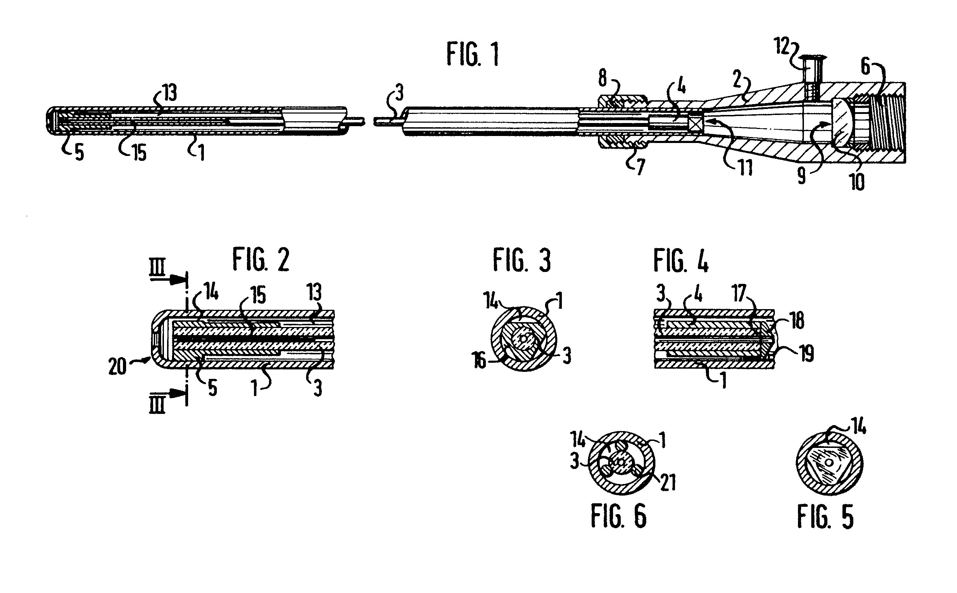

(57) Die Vorrichtung zur Gasspülung eines Wellenleiters für $CO_2$-Laser behandelt einen Wellenleiter, der dünner als ein Keramikhohlleiter (3) ausgebildet ist, der in einem Außenrohr (1) unter Belassen eines Hohlraumes (13) zwischen diesem und dem Keramikhohlleiter geführt ist. Das Außenrohr ist proximalseitig mittels eines eine Fokussieroptik (9) aufnehmenden Anschlußteils (2) mit einem Gasanschluß versehen. Die Führung des Keramikhohlleiters in dem Außenrohr erfolgt mindestens durch je ein proximalseitig und distalseitig angeordnetes Lagerteil (4,5), das sich jeweils örtlich gegenüber der Innenwandung des Außenrohres (1) abstützt. Dabei sind die Lagerteile (4,5) so gestaltet, daß im Bereich der Abstützung freie Fließquerschnitte zur Durchleitung des Spülgases verbleiben. Die Fließquerschnitte sind mit dem lichten Innenquerschnitt des Keramikhohlleiters (3) derart abgestimmt, daß sowohl ein außen als auch innen an dem Keramikhohlleiter (3) vorbeiströmender Gasfluß entsteht, der einerseits eine Kühlung desselben bewirkt und andererseits verhindert, daß Verunreinigungen in das distalseitige Ende des Keramikhohlleiters (3) eindringen (Figur 1).

FIG. 1

Die Erfindung betrifft eine Vorrichtung zur Gasspülung eines Wellenleiters für CO$_2$-Laser, wobei der Wellenleiter als dünner Keramikhohlleiter ausgebildet ist, der in einem vorzugsweise aus Edelstahl bestehenden Außenrohr unter Belassen eines Hohlraumes zwischen diesem und dem Keramikhohlleiter geführt ist, und wobei das Außenrohr proximalseitig mit einem eine Fokussieroptik aufnehmenden und einen Gasanschluß aufweisenden Anschlußteil versehen ist.

In der Medizin werden häufig Laser zur Diagnose und Therapie bzw. zu chirurgischen Zwekken eingesetzt. Dabei hat sich insbesondere in der Gynäkologie in den letzten Jahren der CO$_2$-Laser durchgesetzt und wird hier vor allem laparoskopisch eingesetzt, um z.B. Verwachsungen am Uterus oder an den Ovarien zu beseitigen. Ein besonderer Vorteil dieser Methode liegt im weitgehend unblutigen Arbeiten, da aufgrund der thermischen Wirkung des Laserstrahls kleine Blutgefäße sofort koaguliert werden.

In neuerer Zeit werden als Applikatoren für die CO$_2$-Lasertherapie sogenannte Wellenleiter eingesetzt, die durch eine Trokarhülse z.B. in den Bauchraum einer Patientin eingeführt werden. Solche Wellenleiter bestehen aus einem dünnen Keramikhohlleiter, der aus Stabilitätsgründen in einem Edelstahlrohr gefaßt ist, sowie aus einem Kupplungsteil, das eine Konvexlinse aus Zinkselenid enthält. Die Linse dient dazu, den Laserstrahl auf die Eintrittsöffnung des Keramikhohlleiters zu fokussieren.

Ein Problem bei der Verwendung der Wellenleiter besteht darin, daß sie insbesondere bei hoher Laserleistung und langer Applikationszeit sehr heiß werden können, was eine Gefährdung des Anwenders und der Patientin darstellt und darüber hinaus auch zur Zerstörung des Wellenleiters führen kann. Die Erwärmung des Wellenleiters wird durch die aufgrund der Strahlgeometrie auftretenden Vielfachreflexionen und die damit verbundenen Absorptionen an der Hohlleiterwand verursacht. Des weiteren können bei der Anwendung Körperflüssigkeit sowie bei der Koagulation und beim Schneiden mit dem Laserstrahl entstehender Wasserdampf und Rauchgas in den distalen Bereich des Keramikhohlleiters eindringen. Diese Verunreinigungen absorbieren die Laserenergie besonders stark und können scmit zu einer sehr starken Erhitzung des Wellenleiters und damit zu einer direkten Gefährdung der Patientin führen.

Daher müssen Wellenleiter,um ihren sicheren und effektiven Einsatz zu gewährleisten, intensiv gekühlt und von Verunreinigungen freigehalten werden. Hierzu werden Wellenleiter üblicherweise mittels eines Spülgasanschlusses an CO$_2$-Gasspülsysteme mit einstellbarer Durchflußmenge angeschlossen, wie man z.B. der Gebrauchsanweisung für einen Wellenleiter für ein CO$_2$-Laserübertragungssystem der Firma Heraeus entnehmen kann. Je nach Durchmesser des Wellenleiters werden unterschiedliche Durchflußmengen zum Schutz vor bzw. zum Entfernen von Verunreinigungen benötigt.

Die Freispülung eines Hohlleiters von Verunreinigungen ist beispielsweise aus dem DE-GM 78 10 089 bekannt. Darüber hinaus beschreiben das genannte Gebrauchsmuster sowie das DE-GM 77 09 964 auch vorteilhafte Möglichkeiten der Freispülung des distalen Abschlußelementes bei Verwendung eines Lasers in Verbindung mit einem Linsensystem bzw. einer Laserfaser. Hierbei erfolgt die Freispülung des distalen Abschlußelementes dadurch, daß der Gasstrom am das Linsensystem bzw. die Laserfaser aufnehmenden Führungsröhrchen vorbei zum distalen Ende des Führungsröhrchens geleitet wird. Dazu ist das Führungsröhrchen von einem Außenrohr umgeben und das distale Ende des Außenrohres kann eingebördelt sein, um die Gasströmung auf das distale Abschlußelement umzulenken.

Auf diese Weise lassen sich Verunreinigungen an dem distalen Abschlußelement von Wellenleitern mit optischen Systemen bzw. Verunreinigungen im Inneren von Hohlleitern vermeiden bzw. entfernen. Auch erfolgt auf diese Weise eine Kühlung der Hohlleiter.

Nachteilig beim Einsatz der Wellenleiter nach dem Stand der Technik ist aber, daß bei der Arbeit mit dem Laser in einer Körperhöhle entstehender und die Sicht des Anwenders beeinträchtigender Dampf und Rauch mit den Gasdurchflußmengen, die mit Wellenleitern nach dem Stand der Technik erzielbar sind, nicht ausreichend aus dem Anwendungsgebiet abgeführt werden können. Daher werden bei der Anwendung eines Wellenleiters in Körperhöhlen stets zusätzliche, über einen zusätzlichen Einstich in die Körperhöhle einführbare Gasspülvorrichtungen benötigt, um für eine gute Sicht des Operateurs sorgen zu können.

Es ist daher die Aufgabe der Erfindung, einen Wellenleiter zu schaffen, mit dem es möglich ist, stets für eine freie Sicht im Anwendungsgebiet des Laserstrahls zu sorgen, so daß die zusätzliche Gasspülvorrichtung überflüssig wird, und gleichzeitig die nötige Kühlung des Wellenleiters zu gewährleisten und sicherzustellen, daß der Hohlleiter stets von Verunreinigungen freigespült wird.

Diese Aufgabe wird erfindungsgemäß dadurch gelöst, daß die Führung des Keramikhohlleiters in dem Außenrohr mindestens durch je ein proximalseitig und ein distalseitig angeordnetes Lagerteil erfolgt, die sich jeweils örtlich gegenüber der Innenwandung des Außenrohres abstützen und im Bereich der Abstützung mit freien Fließquerschnitten zur Durchleitung des Spülgases versehen sind.

Dabei kann der Gasanschluß im Bereich zwischen der Fokussieroptik und dem proximalen Ende des Keramikhohlleiters angeordnet sein, und die Fließquerschnitte können im Bereich der Lagerteile in Abstimmung mit dem lichten Querschnitt des Keramikhohlleiters so dimensioniert sein, daß ein Gasfluß sowohl in dem letzten als auch in dem Hohlraum zwischen dem Keramikhohlleiter und dem Außenrohr zustandekommt.

Auf diese Weise wird der Keramikhohlleiter von dem Gasfluß sowohl innen wie außen durchströmt und damit in ausreichender Weise gekühlt. Gleichzeitig verhindert der Gasfluß im Innern des Keramikhohlleiters das Eindringen von Verunreinigungen in den Hohlleiter. Diese Wirkung kann nach dadurch verstärkt werden, daß das Außenrohr an seinem distalen Ende nach innen eingebördelt ausgeführt ist, so daß der außen über den Keramikhohlleiter strömende Gasfluß auf das distale Ende des Hohlleiters umgelenkt wird. Um zu vermeiden, daß bei nicht idealer Ankuppelung des Laserstrahls in den Hohlleiter eine Beschädigung desselben erfolgt, kann das proximalseitige Ende des Keramikhohlleiters mit einem kegelförmigen Kopfteil versehen sein, das mit einer mit dem inneren Hohlraum des Keramikhohlleiters fluchtenden Bohrung versehen ist.

Die Erfindung ist nachstehend anhand eines in der Zeichnung dargestellten Ausführungsbeispieles näher erläutert. Es zeigen:

Figur 1 einen erfindungsgemäßen Wellenleiter teilweise im Schnitt dargestellt,

Figur 2 einen Längsschnitt durch den distalen Endbereich des Wellenleiters nach Figur 1,

Figur 3 einen Schnitt entlang der Linie III-III nach Figur 2,

Figur 4 einen Längsschnitt durch den proximalen Endbereich des Wellenleiters nach Figur 1,

Figur 5 eine Stirnansicht des Wellenleiters nach Figur 4,

Figur 6 einen Querschnitt entlang der Linie III-III nach Figur 2 einer modifizierten Ausführung des distalen Endbereiches des Wellenleiters.

Der erfindungsgemäße Wellenleiter besteht nach Figur 1 aus einem Außenrohr 1 mit einem Anschlußteil 2 an seinem proximalen Ende und einem in dem Außenrohr 1 geführten Keramikhohlleiter 3. Letzterer wird im Bereich seiner Enden durch ein proximalseitig und ein distalseitig angeordnetes Lagerteil 4 bzw. 5 so geführt, daß der Keramikhohlleiter 3 und das Außenrohr 1 zueinander koaxial und beabstandet verlaufen.

Das Anschlußteil 2 dient der optischen Ankuppelung des Keramikhohlleiters 3 an den Spiegelgelenkarm eines Lasersystems und weist hierzu proximalseitig einen Ankuppelbereich 6 auf, der auch mit einem zum Spiegelgelenkarm des jeweils verwendeten Lasersystems passenden Adapter verbindbar ist. Die Verbindung des Anschlußteils 2 mit dem Außenrohr 1 kann durch eine Überwurfmutter erfolgen, die ein mit dem Außenrohr fest verbundenes Teil 8 übergreift. Durch das Teil 8 ist gewährleistet, daß die proximale Eintrittsöffnung des Hohlleiters im Fokus der Linse 10 liegt.

Das Anschlußteil 2 enthält eine Fokussieroptik 9, die aus einer in diesem Teil gasdicht festgelegten Konvexlinse 10, z.B. aus Zinkselenid besteht, und die dazu dient, den Laserstrahl auf die proximale Öffnung 11 des Keramikhohlleiters 3 so zu fokussieren, daß der Laserstrahl mit nahezu parallel zur geometrischen Achse des Keramikhohlleiters ausgerichteter optischer Achse in die Öffnung 11 des Keramikhohlleiters 3 eintreten kann. Das Anschlußteil 2 ist weiter mit einem Gasanschluß 12, z.B. einem Luer-Anschluß versehen, der in den Raum zwischen öffnung 11 des Keramikhohlleiters 3 und Konvexlinse 10 mündet und mit einer nicht gezeigten Gasversorgungseinrichtung zur Gasspülung und Kühlung des Wellenleiters verbindbar ist.

Zur zentrischen Lagerung des Keramikhohlleiters 3 in dem Außenrohr 1 und um einen Hohlraum 13 zwischen Keramikhohlleiter 3 und Außenrohr 1 freizuhalten, ist der Keramikhohlleiter 3 mit mindestens zwei Lagerteilen 4 bzw. 5 versehen, von denen eines am distalen Ende und eines am proximalen Ende des Hohlleiters 3 angebracht ist. Die Lagerteile 4 und 5 sind so ausgestaltet, daß sie den Keramikhohlleiter 3 aufnehmen und im Außenrohr 1 in einer Lage fixieren, bei der Hohlräume 14 zwischen dem Außenrohr 1 und den Lagerteilen erhalten bleiben. Der zur Freihaltung der Sicht im Applikationsgebiet nötige Gasfluß kann somit durch die Hohlräume 13 und 14 hindurchgeleitet werden. Gleichzeitig kann der zur Kühlung des Hohlleiters 3 und zur Freihaltung von Verunreinigungen desselben nötige Gasfluß durch den inneren Hohlraum 15 des Hohlleiters 3 aufrechterhalten werden.

Bei vorgegebenem Innen- und Außendurchmesser von Keramikhohlleiter 3 und Außenrohr 1 läßt sich der Strömungswiderstand im Hohlraum 13 sowohl durch die Anzahl der Lagerteile als auch durch die axiale Außdehnung derselben sowie durch die Änderung der Hohlräume 14 verändern. Hierdurch ist es möglich, das Verhältnis der Gasflüsse durch die beiden Hohlräume 13 und 15 so zu variieren, daß stets auch der zur Kühlung und Freispülung des Hohlleiters 3 von Verunreinigungen erforderliche Gasstrom durch den inneren Hohlraum 15 gewährleistet werden kann.

Die Lagerteile 4 und 5 sind mit dem Keramikhohlleiter 3 z.B. durch Kleben fest verbunden, wobei die Klebestelle des proximalen Lagerteiles 4 in vorteilhafter Weise von der proximalen Öffnung 11, also der bei Betrieb des Wellenleiters sehr heiß werdenden Einkoppelstelle des Laserstrahls, entfernt angeordnet wird.

Um Beschädigungen des Keramikhohlleiters 3 durch Zug- und Druck-Spannungen auszuschließen, die als Folge von Temperaturschwankungen auftreten können, wird nur das proximale Lagerteil 4 fest mit dem Außenrohr 1 verbunden. So kann der Keramikhohlleiter 3 mit dem an ihm befestigten distalen Lagerteil 5 bzw. eventuell weiteren, zwischen distalem und proximalem Ende des Keramikhohlleiters 3 befestigten Lagerteilen bei Temperaturschwankungen die nötigen Längenausdehnungen im Außenrohr 1 ausführen, womit Zug- und Druckspannungen abgebaut werden.

Grundsätzlich können das distale und das proximale Lagerteil 4 bzw. 5 und eventuell weitere Lagerteile so wie in den Figuren 2 und 3 dargestellt ausgebildet sein. Wie man den genannten Figuren entnehmen kann, ist das Lagerteil z.B. ein Drehteil mit einem dem Innendurchmesser des Außenrohres 1 entsprechendem Durchmesser, das mit abgeschrägten Flächen 16 und einer durchgehenden Bohrung versehen ist. Der Durchmesser der Bohrung entspricht dabei dem Außendurchmesser des Hohlleiters 3 und dient zur Aufnahme desselben.

Es kann vorkommen, daß der Laserstrahl an der Einkoppelstelle die Öffnung 11 des Hohlleiters 3 nicht ideal trifft, sondern bei Verwendung eines Lagerteils nach Figur 2 als proximales Lagerteil 4 auf die Stirnfläche 17 des Hohlleiters 3 auftrifft und diesen zerstört. Daher wird als proximales Lagerteil ein wie in den Figuren 4 und 5 dargestelltes Lagerteil 4 mit abgeschrägten Flächen 16 verwendet, bei dem die proximale Stirnfläche 17 durch ein Kopfteil 18 überdeckt und damit vor dem Laserstrahl geschützt wird. Das Lagerteil 4 enthält ebenfalls eine Bohrung, die jedoch abgesetzt ist, das heißt über eine Teillänge des Lagerteils 4 einen Durchmesser aufweist, der am Außendurchmesser des Keramikhohlleiters 3 entspricht und diesem zur Aufnahme dient, und die sich über die Restlänge mit einem Durchmesser fortsetzt, der dem lichten Innendurchmesser des Hohlleiters 3 entspricht. Weiter ist das Kopfteil 18 des Lagerteiles 4 proximal mit einer Kegelfläche 19 ausgestattet. Dadurch werden Laserstrahlen, die nicht in den Hohlleiter 3 gelangen, sondern auf die proximalseitigen Stirnflächen 19 des Kopfteils 18 auftreffen, so reflektiert, daß sie nicht in den Spiegelgelenkarm des Lasersystems zurückreflektiert werden. Bei senkrecht zur Wellenleiterachse gerichteten Flächen 19 könnten die über den Spiegelgelenkarm zurück in das Lasersystem reflektierten Laserstrahlen zu Störungen im Lasersystem führen, die zur Abschaltung des Lasers führen könnten.

Durch die mit Hilfe der genannten Lagerteile 4 und 5 erreichte, beabstandete Anordnung des Keramikhohlleiters 3 im Außenrohr 1 wird nicht nur erreicht, daß bei der Anwendung des Lasers durch den Gasfluß im äußeren Hohlraum 13 stets für eine gute Sicht im Anwendungsgebiet gesorgt werden kann, sondern es wird durch diesen Gasfluß auch die Kühlung des Hohlleiters 3 wesentlich verbessert. Gleichfalls wird durch diesen äußeren Gasstrom die Entfernung von Verunreinigungen am distalen Hohlleiterende wesentlich verbessert, vor allem dann, wenn das Außenrohr 1 an seinem distalen Ende 20 eingebördelt ist, wodurch eine hohe Strömungsgeschwindigkeit des Spülgases an der Strahlaustrittsstelle erreicht wird.

Wie beschrieben, wird das Verhältnis der Gasströme in den beiden Hohlräumen 13 und 15 durch die Anzahl und die Ausgestaltung der Lagerteile je nach verwendetem Keramikhohlleiter 3 variiert, um den für die Kühlung des Hohlleiters 3 erforderlichen Gasfluß durch den inneren Hohlraum 15 sicherzustellen. So kann ein Lagerteil z.B. auch wie in Figur 5 dargestellt ausgebildet sein, wo die Zentrierung des Keramikhohlleiters 3 durch an diesem befestigte Abstandsdrähte 21 erfolgt.

## Patentansprüche

1. Vorrichtung zur Gasspülung eines Wellenleiters für $CO_2$-Laser, wobei der Wellenleiter als dünner Keramikhohlleiter ausgebildet ist, der in einem vorzugsweise aus Edelstahl bestehenden Außenrohr unter Belassen eines Hohlraumes zwischen diesem und dem Keramikhohlleiter geführt ist, und wobei das Außenrohr proximalseitig mit einem eine Fokussieroptik aufnehmenden und einen Gasanschluß aufweisenden Anschlußteil versehen ist, dadurch gekennzeichnet, daß die Führung des Keramikhohlleiters (3) in dem Außenrohr (1) mindestens durch je ein proximalseitig und ein distalseitig angeordnetes Lagerteil (4 bzw.5) erfolgt, die sich jeweils örtlich gegenüber der Innenwandung des Außenrohres (1) abstützen und im Bereich der Abstützung mit freien Fließquerschnitten zur Durchleitung des Spülgases versehen sind.

2. Vorrichtung nach Anspruch 1, dadurch gekennzeichnet, daß der Gasanschluß (12) im Bereich zwischen der Fokussieroptik (9) und dem proximalen Ende des Keramikhohlleiters (3) angeordnet ist und daß die Fließquerschnitte im Bereich der Lagerteile (4 bzw. 5) in Abstimmung mit dem lichten Querschnitt des Keramikhohlleiters (3) so dimensioniert sind, daß ein Gasfluß sowohl in dem letzteren als auch in dem Hohlraum (13) zwischen dem Keramikhohlleiter (3) und dem Außenrohr (1) zustandekommt.

3. Vorrichtung nach Anspruch 1 oder 2, dadurch gekennzeichnet, daß das Außenrohr (1) an seinem distalen Ende (20) nach innen eingebördelt ausgeführt ist.

4. Vorrichtung nach einem der Ansprüche 1 bis 3, dadurch gekennzeichnet, daß das proximalseitige Ende des Keramikhohlleiters (3) mit einem kegelförmigen Kopfteil (18) versehen ist, das eine mit dem inneren Hohlraum des Keramikhohlleiters (3) fluchtende Bohrung aufweist.

FIG. 1

FIG. 2

FIG. 3

FIG. 4

FIG. 5

FIG. 6